# EUROPEAN PATENT SPECIFICATION

(11) **EP 3 380 255 B1**
(45) Date of publication and mention of the grant of the patent: **16.09.2020**
(21) Application number: 16808877.1
(22) Date of filing: 22.11.2016
(51) Int. Cl.: B05C 17/01

(54) **A DISPENSER AND A METHOD OF DISPENSING A PASTY MATERIAL**
SPENDER UND VERFAHREN ZUR ABGABE EINES PASTÖSEN MATERIALS
DISTRIBUTEUR ET PROCÉDÉ DE DISTRIBUTION D'UN MATÉRIAU PÂTEUX

(30) Priority: 27.11.2015 EP 15196651
(43) Date of publication of application: 03.10.2018
(73) Proprietor: 3M Innovative Properties Company, Saint Paul, Minnesota 55133-3427 (US)
(72) Inventor: PAUSER, Helmut, D-82229 Seefeld (DE); PEUKER, Marc, D-82229 Seefeld (DE); BERTL, Mathias, D-82229 Seefeld (DE); BROYLES, Bruce R., Saint Paul, Minnesota 55133-3427 (US)
(74) Representative: Vossius & Partner Patentanwälte Rechtsanwälte mbB
(86) International application number: PCT/US2016/063266
(87) International publication number: WO 2017/091551

(56) References cited:
- WO-A1-2014/179219
- US-A1- 2014 197 195

## Description

### Field of the Invention

The invention relates to a dispenser for a pasty material, in particular for a dental composite material. Further the invention relates to a method of dispensing a pasty material.

### Background Art

A document in the art is exemplified by WO-A-2014/179219.

Dental materials, in particular dental materials used for filling a cavity of a tooth or for fixing a dental restoration on a tooth stump, are often provided in syringes which allow the dispensation several desired amounts for several dental treatments.

In particular dental composite filling materials often are composites containing a predominant amount of fillers, for example quartz, and a relatively small amount of a flowable monomer. Such composites typically further contain a photo initiator allowing the monomer to be polymerized through light energy. Therefore the dental composite filling material may be applied in a cavity in a patient's tooth and light-cured after application in the patient's tooth. Typically such dental composite filling materials exhibit a relatively high viscosity and therefore typically require high forces for dispensing from a dispensing device.

A dispensing device for dispensing dental composite filling materials is for example disclosed in US 7,128,246 B2. Such a device stores a portion of a dental composite filling material which can be extruded by displacing a piston within the device. The device can be coupled to a hand-held dispensing gun providing a relatively high force for displacing the piston via a hand operable leverage. An alternative dispensing device for dental composite filling materials is disclosed in U.S. Design Patent Des. 419,236.

WO 2006/108085 A2 discloses a syringe delivery system for dispensing a highly viscous material through a syringe delivery opening. The system includes a syringe barrel having a delivery opening, a plunger including a threaded shaft that threadably engages the syringe barrel for selectively dispensing a viscous material through the delivery opening, and a plunger gripping member in gripping communication with the plunger that includes means for sealing the threaded shaft of the plunger so as to prevent contamination by foreign matter. The syringe has a sheath that covers the threaded shaft of the plunger so as to hide the plunger beneath the sheath. The sheath provides a sealed environment for the threaded shaft so as to prevent entrance or contamination by foreign matter.
Although a variety of dispensers for dental composite filling materials are available there is still a need for a dispenser which is easy to use, which accounts for hygiene requirements in dentistry and which helps maximizing shelf life of the dental material stored therein.

### Summary of the Invention

The invention relates to a dispenser, particularly for a pasty material. The dispenser comprises a container for the material, a cover and a handle. The dispenser is operable by twisting the cover and the handle about a rotation axis relative to each other in a dispensing direction for dispensing material and in an opposite closing direction. The container has an outlet for the material and the cover has a dispensing opening. The container and the cover are arranged relative to each other for a rotation between a closed position, in which the cover closes the outlet, and an open position, in which the dispensing opening opens or overlaps the outlet. A first engagement mechanism is provided for enabling the container and the handle to engage with each other in at least one angular position during twisting in the closing direction. The first engagement mechanism further forms a freewheel mechanism with respect to a rotation in the dispensing direction. The first engagement mechanism comprises a first snap-stop for providing the engagement between the handle and the container and for impeded disengagement when engaged. A second engagement mechanism is provided for enabling the rotation of the container and the cover in an angular range between the closed position and the open position. The second engagement mechanism further restricts a rotation outside that range. The second engagement mechanism comprises a second snap-stop for providing the restriction between the container and the cover with respect to a twisting toward the dispensing direction. The second engagement mechanism provides for an impeded disengagement when engaged in the open position. The first snap-stop provides for an impeding force to be overcome for disengagement and the second snap-stop providing for a snap-in force to be overcome for engagement. The first and second snap-stop are configured such that the impeding force is greater than the snap-in force.

The invention is advantageous in that it provides a dispenser that allows for dispensation of dental material (particularly a dental composite filling material) in the form of a pre-dosed portion. Therefore, the use of a separate instrument for separating (for example cutting or scraping off) a portion of dental composite filling material from the dispenser is not required. Further the dispenser preferably allows for dispensation of the dental material at a precisely controlled manner and particularly may exhibit minimized afterflow (or run on) of dental composite filling material after suspending dispensation. Although a separate instrument may be used for picking up the pre-dosed portion, any intense interaction (like cutting, abrading, scraping) between the instrument and the dispenser can be minimized. This further helps avoiding bringing any particles abraded or cut from the dispenser into the dental composite filling material due to an interaction between the dispenser and the dental instrument. Further the dispenser is advantageous because it helps providing for a convenient handling, precise dosage and hygienic storage of the dental material.

For the purpose of the present specification the term "force" is meant to cover a "torque", particularly if it is referred to rotating components of the dispenser.

The freewheel mechanism preferably provides for a rotatability of the container and the handle in the dispensing direction. Further, the freewheel mechanism provides for the container and the handle to engage with each other in a rotation in the closing direction in at least angular position of a rotation by maximum 360 degrees. Once engaged, the container and the handle are not rotatable relative to each other in the closing direction.

The container is preferably shaped as a sleeve extending along the rotation axis and at a front end being closed by a front portion through which the outlet extends. The container further preferably has an open rear end opposite of the front end.

The cover is preferably shaped as a sleeve extending along the rotation axis and at a front end being closed by a front portion through which the dispensing outlet extends. The cover further preferably has an open rear end opposite of the front end. The cover front portion is generally hemispherical, preferably bullet-shaped.

The cover and the container are preferably dimensioned such that the outside of the container matches with the inside of the cover. Thus, the container and the cover are slidably rotatable relative to each other as permitted by the second engagement mechanism. The cover front portion is preferably generally hemispherical. The outer radius of the hemispherical container front portion preferably corresponds to the inner radius of the hemispherical shape or the inner shape of the cover front portion.

The cover and the container are preferably each formed by one wall of a substantially uniform thickness. This facilitates manufacturing by injection molding.

The sleeve-shaped portion of the cover may extend frustro-conical, tapering toward the front portion. In contrast, the sleeve-shaped portion of the container preferably has a stepped configuration formed by two adjacent cylindrical sections of different diameters. A front section of the two cylindrical sections is arranged adjacent the front portion and a rear section of the two cylindrical sections forms the rear end of the container. The front section is smaller in diameter than the rear section.

The outlet in the container is arranged in an off-center relationship to the rotation axis. The dispensing opening in the cover is preferably also arranged in an off-center relationship to the rotation axis. Preferably, the outlet and the dispensing opening are arranged such that they overlap, preferably fully overlap, in the open position of the dispenser. Therefore, the container and the cover in combination form a rotary slide valve which each other which can be positioned with the outlet and the dispensing opening overlapping (open position of the dispenser) and further with the outlet and the dispensing opening non-overlapping (closed position of the dispenser. The outlet and the dispensing opening preferably extend along a dispensing axis which is inclined with respect to the rotation axis at an angle of more than 0 degrees less than 90 degrees (included angle), preferably about 45 degrees. The rotary slide valve further provides for cutting off any pasty material dispensed from the outlet of the container. Therefore placing the dispenser from the dispensing position in the open position also causes any material protruding from the outlet of the container to be cut off. Any pasty material which exhibits sticky properties further retains at the cover adjacent the dispensing opening and can be removed conveniently by a user.

The handle preferably has an outer non-circular grip portion around the rotation axis. The grip portion also forms a rear end of the handle. The grip portion further has a generally circular flange further to an opposite front end of the handle. The flange is configured to match within the rear end of the cover. In particular, the flange comprises an outward rim and further to toward the rear end of the handle a groove. The cover preferably has an inward projection or rim for mating with the groove of the flange. Thus, the handle and the cover can be rotatably retained with each other.

A "snap-stop" as referred to herein is preferably configured to provide a stop between two movable (in particular rotatable) components at a pre-determined position of the components relative to each other. Such pre-determined position can be referred to a "stop position". The stop particularly prevents a movement (particularly a rotation) of the two components in only one direction from the stop position of the components relative to each other. Further, the snap-stop is preferably configured such that the components positioned at the stop position are impeded against a movement (or rotation) away from the stop. This means that the movement (or rotation) away from the stop is enabled (not prevented), but requires a pre-determined force to be overcome. The pre-determined force to be overcome for a movement (or rotation) away from the stop is referred to as "impeding force" herein. The impeding force is greater than another force (for example friction force) required for moving (or rotating) the two components outside positions within the snap-stop. The snap-stop is preferably configured such that for movement (or rotation) of the components into the snap-stop a snap-in force is required. Accordingly, the snap-stop provides for the two components to snap into place and thus engage with each other once they are positioned in the stop position.

Preferably, the "components" as referred to in the previous paragraph are in one embodiment the cover and the container of the dispenser and in a further embodiment the container and the handle. Both embodiments are preferably present in the dispenser of the invention.

The first snap-stop is preferably provided between the handle and the container. The first snap-stop prevents or stops a rotation of the handle and the container relative to each other upon reaching the at least one angular position in a rotation of the handle and the container relative to each other in the closing direction, while it permits a rotation of the handle and the container relative to each other in the dispensing direction. The first snap-stop therefore forms part of the freewheel mechanism.

It is noted that although the first snap-stop may impede a rotation in the dispensing direction when engaged, the rotation is still permitted but just in consequence of overcoming a certain force for disengaging. The rotation of the handle and the container relative to each other can be caused by the twisting of the cover and the handle about the rotation axis.

The first snap-stop particularly preferably comprises a pawl and a receptacle within which the pawl can be received in a pre-engaged position, in which further movement of the pawl into the receptacle is enabled, and an engaged position, in which the snap-stop is engaged and further movement of the pawl into the receptacle is prevented. The pawl is preferably provided at or by the container and the receptacle is preferably provided at or by the handle. The pawl may particularly be formed by a partial circumferential wall of the container around the rotation axis. Further the receptacle may be formed in at least a partial circumferential wall of the handle around the rotation axis.

The pawl is preferably formed by a structure which protrudes in a direction axially from the container.

In an embodiment the pawl has a first retention element and the receptacle has a second retention element for engaging with each other to provide the impeding against disengagement of the first snap-stop. The first retention element may be formed by a groove or slot formed within the pawl. Further, the second retention element may be formed as a projection or bulge formed on the container wall. The skilled person will recognize that in the alternative the first retention element may be formed by a projection or bulge and the second retention element may be formed by a groove or slot. The first and second retention element are configured for elastically yielding and resetting radially from the rotation axis for engaging and disengaging. The pawl is preferably elastically deflectable in a dimension transverse to the rotation axis. In particular, the pawl preferably has a dimension which provides for a certain resilience so that the first retention element formed on the pawl can elastically yield and reset in a dimension radially of the rotation axis.

The first snap-stop further preferably provides for a snap-in force to be overcome for engagement. The snap-in force of the first snap-stop may be provided by the projection or bulge over which the pawl is required to slide for engaging.

In an embodiment the cover has a cavity in a wall that faces the pawl. The pawl is preferably restricted against a radially outward movement by the wall in the closed position of the dispenser. This means that in the closed position the cavity and the pawl are angularly (about the rotation axis) offset relative to each other in a non-overlapping relationship.

The pawl is preferably further restricted such that the cavity provides a space for the pawl to radially outwardly move in only the open position. Therefore, the first snap-stop is preferably locked against disengagement in positions outside the open position. Nevertheless the disengagement may be impeded in the open position. The cavity preferably forms part of the first snap-lock and helps for controlling (in particular lowering) the impeding force provided by the first snap-stop in the open position.

In a further embodiment the dispenser is provided with an audible click function. The audible click of the click function is preferably triggered in at least one angular position during rotation of the handle and the container relative to each other in the dispensing direction. In a preferred embodiment the click function is triggered in two angular positions spaced by 180 degrees during rotation of the handle and the container relative to each other in the dispensing direction. It has been found that it is relatively convenient to twist the dispenser for about 180 degrees without changing hand positions. Therefore, each twisting without changing hand positions typically allows reaching the next click. This facilitates metering the amount of material dispensed by audible control.

The audible click function is preferably provided by a baffle formed by the handle. The baffle cooperates with the pawl such that during the rotation of the handle and the container relative to each other the baffle and the pawl periodically meet, whereby the baffle elastically deflects the pawl radially inwardly and releases the pawl subsequently. The click is preferably generated in consequence of the pawl running over an edge of the baffle. Preferably, the click is generated by the edge of the baffle "picking" (like a guitar string) an edge of the pawl while the pawl slides over the edge for resetting. Preferably, both the edge of the pawl and the edge of the baffle that generate the click are sharp edges, in particular formed by surfaces which are angled at 90 degrees or less and having nor chamfer or radius. This is in contrast to a pawl providing a click by hitting a surface when reset. In particular, the picking of the baffle provides for a maximized sound level of the click.

The second engagement mechanism preferably comprises a tappet and a detent for disengageable engagement with one another. The tappet may be formed as a radial outward projection from the container, whereas the detent may be formed as a radial inward projection from the cover. The detent preferably forms part of the second snap-stop and provides for an impeding force to be overcome for disengagement of the second snap-stop. In particular, the tappet preferably snaps behind the detent for engagement of the snap-stop.

A third engagement mechanism is preferably provided for impeding a rotation in the closing direction of the container and the cover relative to each other, when the dispenser is in the open position. Therefore, in the open position the second and the third engagement mechanism each, independently, impede a rotation of the container and the cover relative to each other in the closing direction. It has been found, that thus the reliability of the dispenser can be maximized. In particular, the combination of the second and third engagement mechanism provide for the dispenser to reliably re-close and re-open after a previous dispensation of material.

The dispenser of the invention preferably has a screw plunger. The screw plunger and the handle being anti-twist locked with each other but axially displaceable relative to each other. Therefore, the handle preferably has a blind-hole extending from the front end of the handle into the handle. The blind-hole may comprise a guiding ridge extending parallel to the rotation axis and protruding radially inwardly. The screw plunger at a rear end of it may have a corresponding radial groove for slidably receiving with the guiding ridge. Thus, the anti-twist lock, on the one hand, and the axial displaceability, on the other hand, are implemented. In a preferred embodiment the handle comprises three guiding ridges which are uniformly distributed by 120 degrees angular offset relative to each other about the rotation axis. The screw plunger accordingly has preferably three corresponding grooves.

Preferably a screw connection is formed between the container and the screw plunger. The rear section of the container therefore preferably comprises a nut which has an inner thread for cooperating with the thread of the screw plunger. The nut is preferably axially fixed and anti-twist locked, for example mechanically locked, welded or glued, within the container. The thread is preferably a pitch thread, for example having a diameter of between about 2 mm and 10 mm, preferably about 6 mm with a pitch of between about 0.5 mm and 1.5 mm, preferably about 1 mm. The screw plunger at least with a front portion is preferably received within the container. Further, the screw plunger has a seal at the front end for slidably sealing with the inside of the container.

The cover and the handle are preferably attached to one another and entirely enclose the container except for any portion of the container which is exposed by the dispensing opening. Accordingly, any rotation of the container relative to the cover or the handle is controlled by the first and/or second engagement mechanism and caused by a rotation of the handle and the cover relative to each other. In any such rotation the third engagement mechanism may support the function of the second engagement mechanism as described in further detail below.

In an embodiment a notch is provided between the container and the cover. The notch preferably extends over a distance in a dimension parallel to the rotation axis. This means that the notch may extend straight or curved but runs over a distance or length that can be measured in a dimension parallel to the rotation axis. The notch is preferably arranged such that it is located between the outlet and the dispensing opening in the closed position of the dispenser. Preferably, the notch is provided in the container. The notch interrupts a path between the outlet and the dispensing opening on which a liquid could creep from the dispensing opening toward the outlet, for example a disinfectant penetrating through the dispensing opening between the cover and the container due to capillary effects. Thus, contamination of the material stored in the container by a liquid penetrating into the dispenser can be avoided.

In a further embodiment one or more recessed areas are provided in the container to provide a space between the container and the cover. The recessed area(s) is/are located outside a ring-shaped seal around the outlet. Therefore, the ring-shaped seal of the container preferably seals with the cover so that material dispensed from the dispenser is hindered in penetrating between the cover and the container. However, any material that accidentally leaking in an area between the cover and the container can be picked up by the space provided by the recessed area(s). This helps maximizing the reliability of the dispenser because any impact on the forces required to rotate the container and the cover relative to each other by material leaking between the cover and the container can be minimized.

The dispenser is preferably injection molded. In particular, each component of the dispenser (except for the pasty material filled with in the container) is preferably injection molded. The cover, the container and the screw plunger are preferably made of polybutadiene terephthalate (PBT). Optionally the container is made of polypropylene (PP). The handle is preferably made of a styrene acrylonitrile copolymer (SAN) and may be optionally made of polystyrene (PS), polycarbonate (PC) or polyethylene terephthalate (PET).

The dispenser, in particular the container, preferably contain the pasty material. The pasty material is preferably a dental material, in particular a dental composite material, and in more particular a light hardenable dental composite filling material.

Dental composite filling materials are typically characterized by at least one or all of the following features:
a) radiation curable (especially within the region of visible light, in particular blue light);
b) formulated as a one-component composition (in contrast to e.g. dental impression materials which are formulated as two-component compositions which are mixed from a base paste and a catalyst paste shortly prior to use);
c) highly viscous;
d) slightly tacky, for example sticky if touched with the fingers;
e) "cuttable", for example can be cut into pieces with a dental instrument or spatula;
f) "firm" for example shapeable by applying manual forces but generally self-supporting in absence of such forces at least over a time period of about 5 minutes. A combination of the features (a), (b), (c) and (d) is sometimes preferred.

Dental composite materials typically comprise a hardenable resin matrix comprising hardenable components, an initiator system suitable to harden the hardenable components contained in the resin matrix and filler(s). The filler content is typically above about 50, 60 or 70 wt.-% with respect to the weight of the dental composite material. Typical ranges include from about 50 to about 90 wt.-% or from about 60 to about 80 wt.-%. The hardenable components typically comprise unsaturated moieties (carbon-carbon unsaturation) like (meth)acrylate moieties. In order to be crosslinkable, the hardenable components typically comprise at least about 2 unsaturated moieties.

The composite material may comprise only one type of filler or different types of fillers. Suitable filler(s) include fumed silica, quartz, ground glasses, non-water-soluble fluorides such as CaF₂, silica gels such as silicic acid, in particular pyrogenic silicic acid and granulates thereof, cristobalite, calcium silicate, zirconium silicate, zeolites, including the molecular sieves, barium sulphate and/or yttrium fluoride. Suitable fumed silicas include for example, products sold under the tradename AerosilTM series OX-50, -130, -150, and - 200, Aerosil R8200 available from Degussa AG, (Hanau, Germany), CAB-O-SILTM M5 available from Cabot Corp (Tuscola, Ill.), and HDK types, e.g. HDK-H 2000, HDK H15; HDK H18, HDK H20 and HDK H30 available from Wacker. The average surface area of the silica particles is preferably greater than about 15 m²/g more preferably greater than about 30 m²/g.

A "hardenable component or material" or "polymerizable component" is any component which can be cured or solidified e.g. by radiation-induced polymerization. A hardenable component may contain only one, two, three or more polymerizable groups. Typical examples of polymerizable groups include unsaturated carbon groups, such as a vinyl group being present i.a. in a (methyl)acrylate group.
"(Meth)acryl" is a shorthand term referring to "acryl" and/or "methacryl". For example, a "(meth) acryloxy" group is a shorthand term referring to either an acryloxy group (i. e.,CH₂=CH-C(O)-O-) and/or a methacryloxy group (i. e., CH₂=C(CH₃)-C(O)-O-).

A "curing, hardening or setting reaction" is used interchangeable and refers to a reaction wherein physical properties such as viscosity and hardness of a composition changes over the time due to a chemical reaction between the individual components.

"Light hardenable" shall mean that the composition can be cured by applying radiation with light, preferably radiation with light at a wavelength within the visible light spectrum under ambient conditions (for example approximately 23 ± 10 degrees Celsius) and within a reasonable time frame (e.g. within about 15, 10 or 5 minutes).

The term "visible light" is used to refer to light having a wavelength of about 380 to about 750 nanometers (nm).

In a further aspect, the invention relates to a method of dispensing a pasty material. The method comprises the steps of:
- providing a dispenser as described beforehand;
- starting from the closed position, twisting the handle and the cover in the dispensing direction relative to each other and thereby entraining the container by the handle based on an engagement of the container and the handle with each other;
- further twisting the handle and the cover in the dispensing direction relative to each other and thereby reaching the open position in which the cover and the container are engaged for impeded disengagement;
- further twisting the handle and the cover in the dispensing direction relative to each other and causing the container and the handle to disengage from each other;
- further twisting the handle and the cover in the dispensing direction relative to each other and thereby dispensing the material;
- twisting the handle and the cover in the closing direction relative to each other whereby the handle and the container rotate relative to each other;
- further twisting the handle and the cover in the closing direction relative to each other and thereby causing the container and the handle to engage with each other;
- further twisting the handle and the cover in the closing direction relative to each other and thereby causing the container and the cover to disengage from each other; and
- further twisting the handle and the cover in the closing direction relative to each other and thereby reaching the closed position.

During twisting the dispenser from the open toward the closed position any material dispensed is automatically cut or sheared off by the outlet and the dispensing opening moving from an overlapping position to an offset position.

In one embodiment the dispenser has a spring cam. The spring cam may be attached at the container to resiliently urge toward the cover. In particular, the spring cam may be radially movable against spring force but fixed relative to the container against a movement in a circumferential direction. The cover may have a first engagement groove into which the spring cam can engage. Accordingly, the cover and the container may be retained against relative movement to each other when the spring cam engages with the first engagement groove. The handle, or a part that is fixedly connected with the handle, may have a second engagement groove into which the spring cam can move in a situation in which the snap stop of the first engagement mechanism is engaged. Thereby the handle and the container are locked with each other against relative movement to each other. This prevents that the container and the handle can move relative to each other as long as the cover is in an intermediate position between positions in which the dispenser is closed or open. The cover may have a third engagement groove. Upon reaching a position in which the dispenser is open the spring cam snaps into the third engagement groove, thus also retracting from the second engagement groove.

### Brief Description of the Figures

- Fig. 1: is a side view of a dispenser according to an embodiment of the invention;
- Fig. 2: is a side view of the dispenser of Fig 1 in use;
- Fig. 3: is an exploded view of a dispenser according to an embodiment of the invention;
- Figures 4-11: are schematic illustrations of the operation of a dispenser according to an embodiment of the invention;
- Fig. 12: is a perspective partial cross-sectional view of a dispenser according to an embodiment of the invention;
- Figures 13-19: are schematic illustrations of the function of the first engagement mechanism of a dispenser according to an embodiment of the invention;
- Fig. 20: is a perspective partial cross-sectional view of a dispenser according to an embodiment of the invention;
- Fig. 21-23: are schematic illustrations of the operation of a dispenser according to an embodiment of the invention;
- Fig.24: is a perspective partial view a dispenser according to an embodiment of the invention at one stage of operation;
- Fig. 25: is a perspective partial view the dispenser shown in Fig. 24 at a further stage of operation; and
- Fig. 26: is a perspective partial view the dispenser shown in Fig. 24 at still a further stage of operation.

### Detailed Description of the Invention

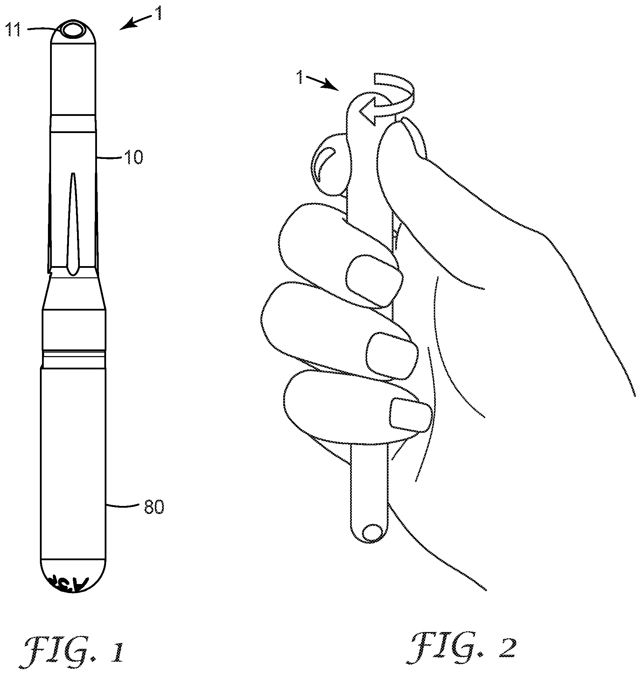

Fig. 1 shows an exemplary dispenser 1 for dispensing a pasty material as it may be used with the present invention. A similar dispenser is disclosed in further detail in WO 2014/179219 A1. The dispenser 1 has a cover 10 and a handle 80. The cover 10 and the handle 80 are rotatably connected to each other and form in combination a housing of the dispenser 1. The cover 10 forms a dispensing opening 11 of the dispenser 1. The dispensing opening 11 forms the only opening in the housing. In this regard any gap eventually formed by the rotatable interconnection between the cover 10 and the handle 80 is not regarded as a opening in the context of the present specification.

The operation of the dispenser 1 is illustrated in Fig. 2. The material stored within the dispenser 1 can be dispensed by rotating the cover 10 and the handle 80 in one direction relative to each other, which is further referred to as "dispensing direction". The dispenser 1 additionally has an automatic opening function and a pressure relief function: A rotation of the cover 10 and the handle 80 in the dispensing direction relative to each other causes the initially closed dispenser to open first and only subsequently causes the material to be urged forward for dispensation upon further rotation in the same direction. The dispenser 1 can be closed again by rotating the cover 10 and the handle 80 in the opposite direction relative to each other, which is further referred to as "closing direction". Thereby a rotation of the cover 10 and the handle 80 in the closing direction relative to each other first causes any pressure from dispensing the material to be relieved and only subsequently causes the dispenser 1 to close. Therefore, the dispenser is adapted such that it opens automatically before any material is urged forward for dispensing and such that any pressure in the material is relieved before the dispenser closes again. This functionality helps preventing unintentional urging of the material forward although the dispenser 1 is still closed and thus avoids leakage of any material beneath the cover 10 inside the dispenser 1. Further, this functionality helps preventing that a pressure in the material is captured within the closed dispenser and thus again avoids leakage of any material beneath the cover 10 as well as it avoids run-on or after-flow of the material prior to closing the dispenser 1.

Accordingly, although the dispenser 1 can be operated by only two operations (rotating in the dispensing direction and rotation in the closing direction) the dispenser is adapted to perform four different functions in a predetermined sequence. Exemplary structures for providing these functions are described in further detail in the following.

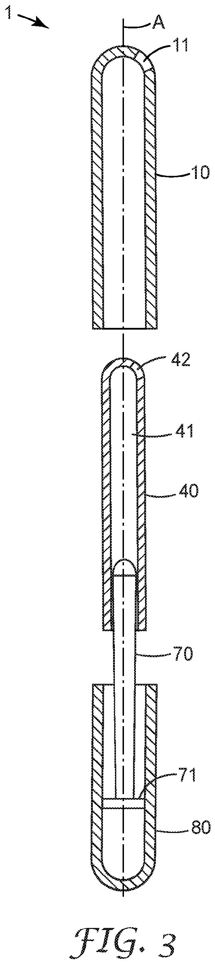

Fig. 3 shows an exploded view of the dispenser 1. The dispenser 1 has a container 40 within which a screw plunger 70 is received. The container 40 forms a material chamber 41 within which the material (not shown) is received. Further, the container 40 has an outlet 42 through which the material can be dispensed by screwing the screw plunger 70 into the container 40. The screw connection between the container 40 and the screw plunger 70 may be implemented based on the concepts disclosed in co-pending European patent application no. EP15177972.5. The screw connection between the container 40 and the screw plunger 70 is such that a rotation of the container 40 and the screw plunger 70 relative to each other in the dispensing direction causes the screw plunger 70 to move into the container 40 and a rotation of the container 40 and the screw plunger 70 relative to each other in the closing direction causes the screw plunger 70 to retract. The rotation of the container 40 and the screw plunger 70 relative to each other in the dispensing direction thus causes the material stored in the container 40 to be pressurized and therefore to be urged forward for dispensation. The rotation of the container 40 and the screw plunger 70 relative to each other in the closing direction however relieves any pressure from the material. As explained in further detail below the dispenser 1 is adapted such that the rotation of the container 40 and the screw plunger 70 relative to each other in the closing direction is limited to a predetermined retraction stroke which is sufficient to relieve any pressure in the material but insufficient to cause any air to be sucked into the container 40.

The screw plunger 70 and the handle 80 are anti-twist locked (against a rotation about rotation axis A) but axially movable (along rotation axis A) with each other. The anti-twist lock between the screw plunger 70 and the handle 80 is bidirectional (in both directions of rotation). Therefore, any rotation of the handle 80 relative to the container 40 also causes the same rotation of the screw plunger 70 relative to the container 40. However, due to the screw connection between the container 40 and the screw plunger 70 the rotation of the handle 80 relative to the container 40 also causes the screw plunger 70 to axially move relative to the container 40. In the example, the anti-twist lock is formed by an elongated rectangular groove along the rotation axis in the handle 80 within which a corresponding rectangular portion 71 of the screw plunger 70 is axially slidably received.

For dispensing the material the opening 11 can be selectively brought in alignment with the outlet 42 by rotation of the cover 10 relative to the container 40. For closing the dispenser 1 the cover 10 and the container 40 can be rotated to misalign the opening 11 and the outlet 42 so that a wall of the cover 10 blocks the outlet 42. Accordingly, the cover 10 and the container 40 form a rotary slide valve in combination.

For opening and dispensation, the dispenser 1 can be operated by rotating the handle 80 and the cover 10 in the dispensing direction relative to each other. The dispenser 1 has a sequence control mechanism (described in detail further below) which provides for a controlled coupling interaction between the handle 80 and the cover 10 via the container 40. In particular, the sequence control mechanism is configured such that, starting with the dispenser when it is closed, a rotation of the handle 80 and the cover 10 in the dispensing direction relative to each other causes the handle 80 to entrain the container 40 due to an engagement between the handle 80 and the container 40. Upon reaching a position in which the outlet 42 and the opening 11 are aligned a stop provided between the cover 10 and the container 40 prevents further relative movement between the cover 10 and the container 40. The engagement between the handle 80 and the container 40 is configured such that further rotation of the handle 80 and the cover 10 in the dispensing direction relative to each other causes the engagement to disengage so that the handle 80 can freely rotate relative to the container 40 but with the container 40 and the cover 10 fixed (not rotating) relative to each other. As described above the rotation of the handle 80 and the container 40 relative to each other causes material to be dispensed.

The sequence control mechanism is further configured such that, starting with the dispenser 1 when it is open, a rotation of the cover 10 and the container 40 in the closing direction relative to each other is impeded by a further engagement so that the handle 80 and the container 40 initially rotate relative to each other in the closing direction while the cover 10 and the container 40 not yet rotate (being still fixed) relative to each other. Upon reaching a position in which the outlet 42 and the opening 11 are fully misaligned a stop provided between the handle 80 and the container 40 prevents further relative movement between the handle 80 and the container 40. A further urging of the handle 80 and the container 40 relative to each other therefore causes the handle 80 to entrain the container 40 so that both, the handle 80 and the container 40 rotate together. The impeding engagement between the cover 10 and the container 40 is configured such that further rotation of the handle 80 and the cover 10 in the closing direction causes the cover 10 and the container to disengage in consequence of the handle 80 entraining the container 40. Thus, the cover 10 and the container 40 rotate relative to each other so that the dispenser 1 closes.

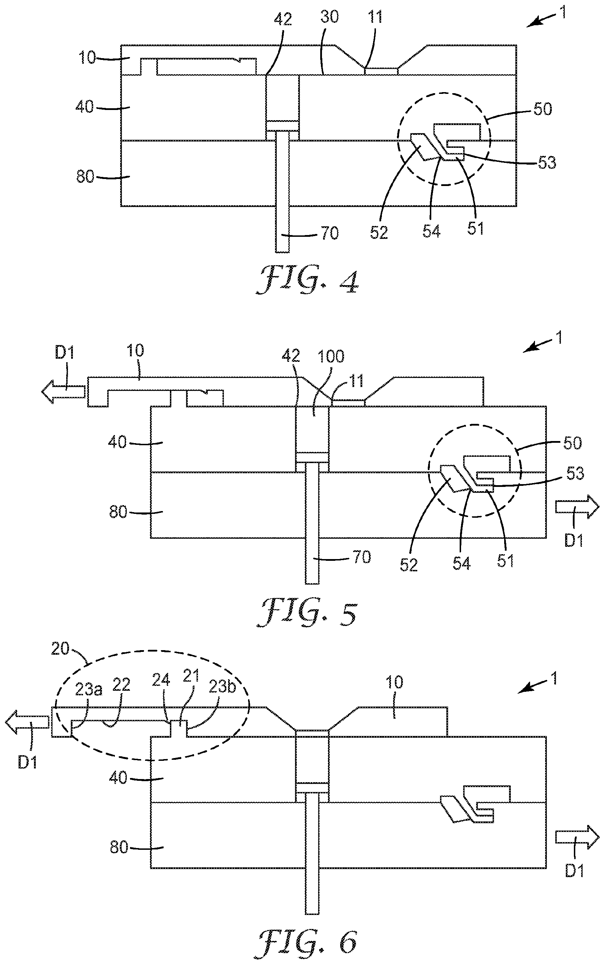

Figures 4 to 11 illustrate the function dispenser 1 and the sequence control mechanism of the dispenser 1 at different stages. In these Figures the dispenser 1 is illustrated strongly abstracted and in particular with the cover 10, the container 40 and the handle 80 being presented in an unwound view. This means that, for better explanation purposes only, the walls of the cover 10, the container 40 and the handle 80 each are illustrated planar (unwound) although these walls are circular circumferential walls in reality. Further, some details of the cover 10, the container 40 and the handle 80 are omitted and the scale is purely schematic.

In Fig. 4 the dispenser 1 is illustrated in an initial stage. In the initial stage the dispenser 1 is closed. A new dispenser 1 is for example typically provided by the manufacturer in the initial stage. In this initial stage the dispensing opening 11 is displaced from the outlet 42 so that the dispensing opening 11 and the outlet 42 do not overlap. Further, the container 40 is in contact with the dispensing opening 11, whereas the cover 10 is in contact with the outlet 42. Thus, the cover 10 closes or seals the outlet 42. As shown in this example, in an area 30 formed between the cover 10 and the container and between the outlet 42 and the dispensing opening 11 the container 40 and the cover 10 are in sliding contact with each other.

In Fig. 5 the cover 10 and the handle 80 are moved (in reality rotated) in the dispensing direction as indicated by the arrows D1. The force for causing the movement of the cover 10 and the handle 80 relative to each other is only applied to the cover 10 and the handle 80, whereas the container 40 is entrained by either the cover 10 or the handle 80 as the control mechanism provides for. Without the control mechanism the container 40 would be entrained by either the cover 10 and/or the handle 80 depending on the friction between the cover 10 and the container 40 and the handle 80 and the container 40 so that the movement of the container 40 would be arbitrary. However, at the stage shown the control mechanism provides for the container 40 to be entrained by the handle 80 so that there is no relative movement between the container 40 and the handle 80, while the cover 10 and the container 40 move relative to each other. This is achieved by a first engagement mechanism comprising a first snap-stop 50. The first snap-stop 50 provides for an engagement between the handle 80 and the container 40. Further the first snap-stop 50, when engaged, prohibits a relative movement between the handle 80 and the container 40 in a direction opposite of the dispensing direction D1, but just impedes a disengagement when the handle 80 and the container 40 are urged relative to each other toward the dispensing direction D1. Accordingly, at this stage during moving the handle 80 and the cover 10 relative to each other in the dispensing direction D1, there is a friction force between the cover 10 and the container 40 as well as a friction force between the handle 80 and the container 40 plus an impeding force provided by the first snap-stop. The friction force and the impeding force between the handle 80 and the container 40 together form a total pulling force. It is noted that the friction force may include forces that result from a friction between the screw plunger and the container and/or the screw plunger and the material with which the screw plunger is in touch. The first snap-stop 50 is configured such that the pulling force between the handle 80 and the container 40 is sufficient to prevail over the friction force between the cover 10 and the container 40. The total pulling force further preferably accounts for any tolerances of the friction force, for example as they may be caused by dirt or pasty material getting between the cover 10 and the container 40. In the example, the first snap-stop 50 comprises a pawl 51 and a recess 52 for receiving the pawl 51. The recess 52 resembles the shape of a "garage" for the free end of the pawl 51 and therefore is further referred to as "garage" herein. The garage 52 is provided at the handle 80, whereas the pawl 51 is provided at the container 40. In more particular, the pawl 52 can enter the garage 52 from only one side and is stopped by a garage end wall 53 upon further movement. Further, the pawl 51 can be disengaged from the garage 52. However, a retention element 54 in the garage 52 (at the handle 80) impedes the disengagement. Upon engaging the garage 52 the pawl 51 further snaps behind the retention element 54.

At the stage shown the dispensing opening 11 and the outlet 42 are not yet overlapping so that the dispenser 1 is not yet open. Due to the fact, that there is no relative movement between the container 40 and the handle 80 up to this stage, the screw plunger 70 has not displaced axially yet. Therefore the pasty material 100 is not yet urged toward the cover 10. This prevents the pasty material from being urged between the cover 10 and the container 40.

In Fig. 6 the dispensing opening 11 and the outlet 42 are fully aligned so that the dispenser 1 is open. Further, the dispenser 1 has a second engagement mechanism which comprises a second snap-stop 20. The second snap-stop 20 comprises a tappet 21 provided at the container 40 and a recess 22 provided in the cover 10. The tappet 21 is restricted for a movement only within the recess 22. This is because end walls 23a, 23b block the tappet from any movement outside the recess 22. Therefore the second engagement mechanism enables the movement (rotation) of the container 40 and the cover 10 in an angular range and restricts or prohibits a movement (rotation) outside that range. The second engagement mechanism further has a detent 24 behind which the tappet has snapped. The detent 24 impedes the tapped so that a movement of the cover 10 and the container 40 in the opposite direction of the dispensing direction is also impeded.

At the stage shown the tappet 21 and the wall 23b of the recess 22 are in contact and prohibit a further relative movement of the container 40 and the cover 10 in the dispensing direction D1. Upon, however, further urging the handle 80 and the cover 10 in the dispensing direction the first engagement mechanism disengages.

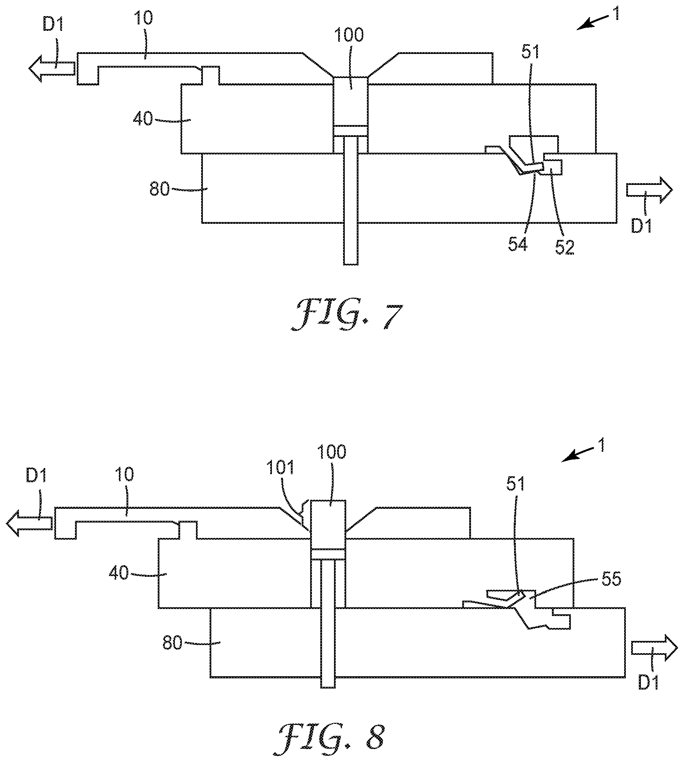

This is illustrated in Fig. 7. As shown, the pawl 51 has overcome the retention element 54 and is retracted from the garage 52. Because in the open position of the dispenser 1 as shown any relative movement in the dispensing direction D1 between the cover 10 and the container 40 is blocked, urging the handle 80 and the cover 10 relative to each other in the dispensing direction D1 causes the handle 80 and the container 40 to move relative to each other in the dispensing direction D1. Therefore, also the screw plunger 70 is moved relative to the container 40 in the dispensing direction D1 and thus screws into the container and urging the material 100 toward the dispensing opening 11.

In Fig. 8 the dispenser 1 is operated further in the dispensing direction D1 so that a strand 101 of material 100 is dispensed from the dispensing opening 11. The pawl 51 is entirely removed from the garage 52 and accommodated in a space 55 provided at the container 40. As can be seen from the illustration, the handle 80 and the container 40 can be freely rotated in the dispensing direction D1 without the first engagement mechanism re-engaging. Therefore, a user can dispense any desired amount of pasty material.

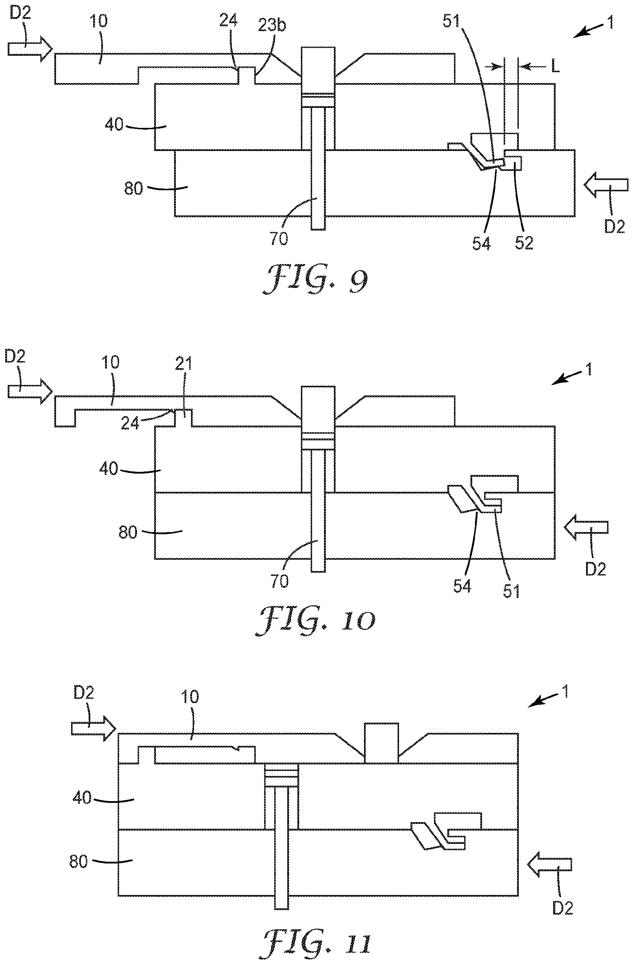

Fig. 9 illustrates a stage in which no further material is dispensed and in which the cover 10 and the handle 80 are moved relative to each other in the closing direction D2. At this stage the dispenser 1 is still in the open position and the cover 10 and the container 40 are impeded against a relative movement in the closing direction D2. This is provided by the detent 24 which hinders the tappet 21 in moving away from the wall 23b. During a movement of the handle 80 and the cover 10 relative to each other in the closing direction the second engagement mechanism provides for the container 40 to be entrained by the cover 10 while the container 40 and the handle 80 move relative to each other until the first engagement mechanism engages. At the stage shown the pawl 51 is just about to enter the garage 52 but is not yet snapped behind the retention element 54. Due to the garage 52 having a length L along which the pawl 51 travels to engage, the garage 52 provides for a minimum pre-determined distance at which the container 40 and the handle 80 can be moved relative to each other in the closing direction D2 at any position of the container 40 and the handle 80 relative to each other in the open position of the dispenser 1. This movement of the container 40 and the handle 80 relative to each other in the closing direction D2 further causes the screw plunger 70 to be retracted in a direction away from the dispensing opening 11. Thus any pressure in the material that may exist from dispensing can be relieved while the dispenser is in the open position. Any residual pressure in the dispenser 1 can thus be minimized. Further, any leakage of the material, for example in the area between the cover 10 and the container 40, can be minimized.

Fig. 10 shows the dispenser 1 at a stage in which the pawl 51 is snapped behind the retention element 54 so that the first engagement mechanism is engaged. During a movement from the stage shown in Fig. 9 to the stage shown in Fig. 10 the pawl 51 overcomes the retention element 54. The associated snap-in force required for the pawl 51 to snap over the retention element 54 is transmitted via the second engagement mechanism, in particular via the detent 24 that impedes the tappet 21. The associated impeding force to be overcome so that the tappet 21 disengages from the detent is greater than the snap-in force. This is provided by the shape and dimensioning of the detent 24 and tappet 21, on the one hand, and by the shape and dimensioning of the engagement element 54 and the pawl, on the other hand. In the example, the detent 24 has a flank to be overcome by the tappet that is steeper than a flank of the retention element 54 to be overcome by the pawl 21. Other possibilities exist to provide a pre-determined difference between the impeding force of the second engagement mechanism and the snap-in force of the first engagement mechanism. Once the first engagement mechanism is engaged a further movement of the container 40 and the handle 80 in the closing direction is prohibited so that such a further movement causes the second engagement mechanism to disengage. At this stage the screw plunger 70 is retracted over at least minimum pre-determined distance so that the pressure in the dispenser 1 is minimized.

Upon disengagement of the second engagement mechanism and a further movement of the handle 80 and the cover 10 relative to each other in the closing direction D2, the dispenser 1 is brought in the closed position as illustrated in Fig. 11. Thereby the container 40 is entrained by the handle 80.

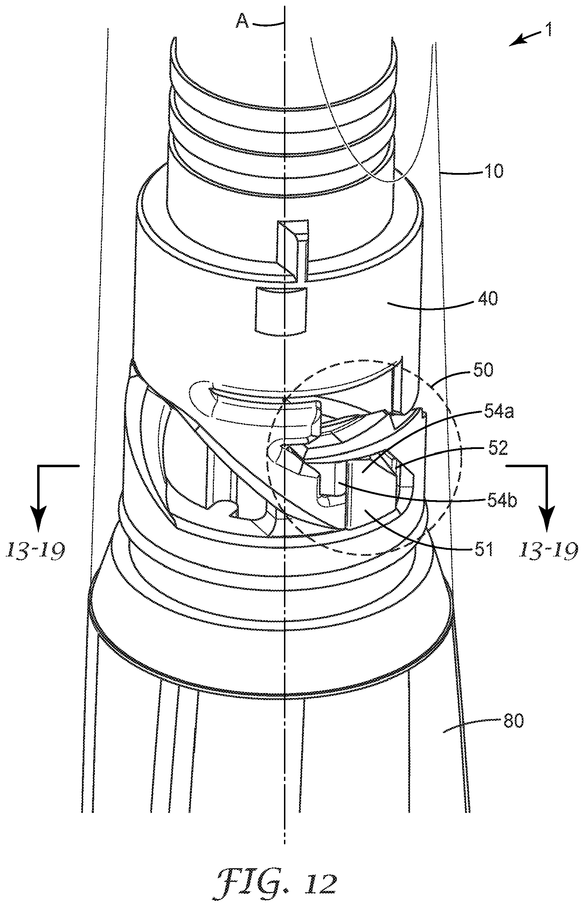

Fig. 12 shows a portion of the dispenser 1 with the snap-stop 50 and the pawl 51 and the garage 52 in more detail. In contrast to the schematic illustrations of Figures 4 to 11 the pawl 51 of the real embodiment moves radially relative to the rotation axis A for engaging with and disengaging from the garage 52 and/or the retention element (not visible in this view). The skilled person will however recognize that the pawl may likewise function based on an axial movement with respect to the rotation axis A as illustrated in Figures 4 to 11. It has however been found that the radial movement allows for a design of the pawl 51 in which the magnitude of the movement can be minimized while the strength of the stop function of the snap-stop 50 is maintained. This minimizes further the mechanical stress the pawl is exposed to and therefore helps maximizing the reliability of the dispenser 1. In the example, the snap-stop 50 has a first retention element 54a and a second retention element 54b, as further described in the following.

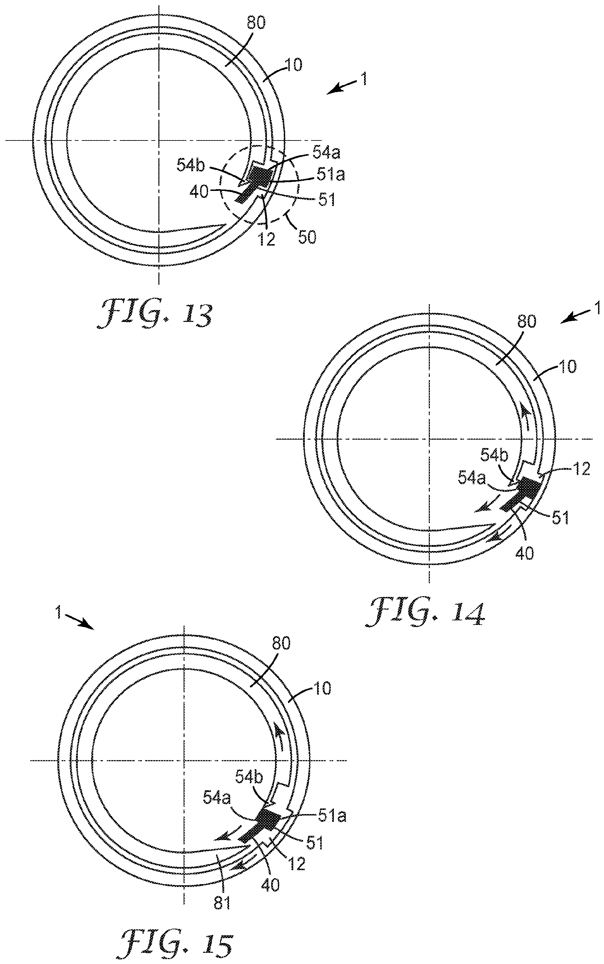

Figures 13 - 18 illustrate in a cross-sectional view (as indicated in Figure 12) a rotation of the handle 80 and the container 40 relative to each other in the dispensing direction.

Fig. 13 illustrates the dispenser in the open position (compare to Fig. 6). In particular, at the stage shown the pawl 51 of the container 40 (only the pawl 51 of the container 40 being visible) is snapped with the first retention element 54a of the pawl 51 behind the second retention element 54b of the handle 80. At this stage a twisting of the handle 80 and the cover 10 relative of each other in the dispensing direction causes the first engagement mechanism 50 to disengage as illustrated in Fig. 14. This is because the second engagement system (provided between the cover 10 and the container 40 and being not visible) causes the container 40 to be entrained by the cover 10 (compare to Fig. 7). The disengagement of the first and second retention element 54a, 54b is enabled by the cavity 12 formed on the inside of the cover 10. The cavity 12 is sized to extend over only a part of the circumference of an inner wall of the cover 10 about the rotation axis. In the open position of the dispenser 1 the cover 10 and the container 40 are positioned such that the cavity 12 is located to enable the pawl 51 to move into the cavity 12. The pawl 51 in the example particularly has a protrusion 51a which can move into the cavity 12.

At the stage shown in Fig. 15 the retention elements 54a, 54b are fully disengaged. Further twisting of the handle 80 and the cover 10 relative of each other in the dispensing direction therefore causes the pawl 51 to be deflected radially inwardly by a baffle 81 formed by a wall of the handle 80.

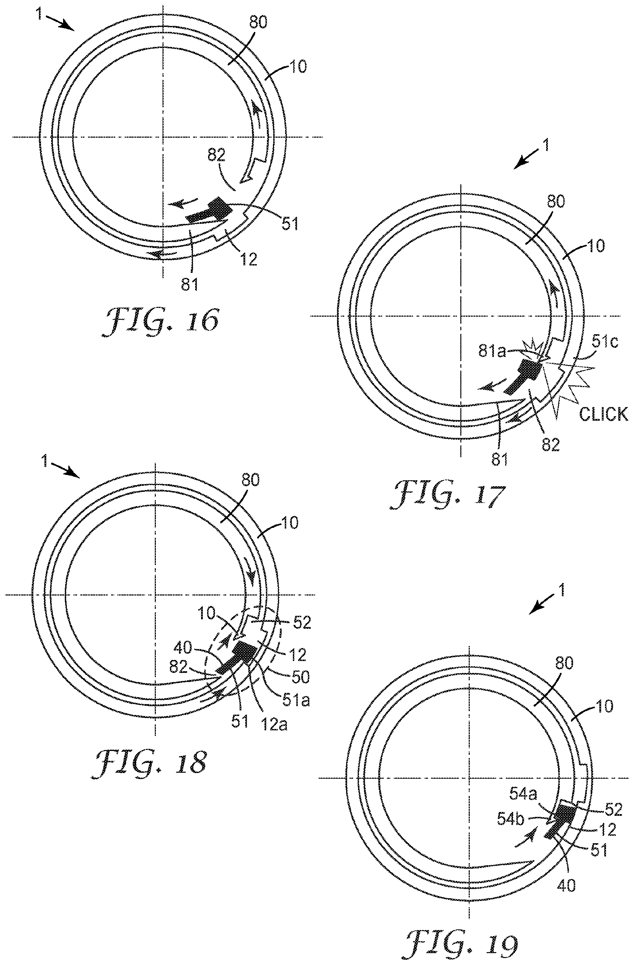

This is shown in Fig. 16. The baffle 81 is formed partly circumferentially about the rotation axis A (extending perpendicular to the area of the Figure), and also comprises the recess 52 of the first engagement mechanism 50. As the handle 80 and the cover 10 are further twisted relative to each other in the dispensing direction (compare to Fig. 8) the pawl 51 is maintained deflected and under a pretension while the handle 80 and the container 40 rotate relative to each other. The container 40 thereby is prevented from rotating relative to the cover 10 by the second engagement mechanism (not shown). Accordingly, twisting the handle 80 and the cover 10 relative to each other in the dispensing direction causes material to be dispensed.

As shown in Fig. 17 the baffle 81 extends circumferentially but leaves a window 82 into which the pawl 51 snaps back radially outwardly. At the illustrated stage due to the pretension, the pawl 51 makes a noise (audible as a "click") as it runs over the edge 81a of the baffle 80 to snap into the window 82. It has been found that the edge 81a should be formed as a sharp edge rather than a rounded edge to maximize the level of the sound. Further, a corresponding edge 51c of the pawl 51, which cooperates with the edge 81a for generating the sound, is likewise sharp rather than rounded. In the example the audible click occurs once per round (per 360 degrees rotation angle) in a rotation of the handle 80 and the container 40 relative to each other in the dispensing direction. In another example the audible click occurs twice per round (per 180 degrees rotation angle) in a rotation of the handle 80 and the container 40 relative to each other in the dispensing direction. In such an example the baffle leaves two windows, although only one first snap-stop may be provided. Because, the angle of rotation of the handle 80 and the container 40 relative to each other is proportional (due to the screw plunger being directly rotated by the handle 80) to an amount of material dispensed from the dispenser a user can meter the amount by help of the number of clicks that occur during dispensation.

Fig. 18 illustrates a stage at which - relative to the situation shown in Fig. 17 - the pawl 51 is snapped into the window 82. This stage corresponds to the stage shown in Fig. 15. At this stage it is possible to dispense further material by twisting the handle 80 and the cover 10 relative to each other in the dispensing direction (as illustrated in Figures 16 and 17). Alternatively, at this stage the handle 80 and the cover 10 can be twisted relative to each other in the closing direction for discontinuing dispensation and for re-closing the dispenser 1. Further, at this stage the protrusion 51a extends into the cavity 12 and a cavity edge 12a engages the protrusion 51a. Thus, a third engagement mechanism is provided. This third engagement mechanism impedes a rotation of the container 40 and the cover 10 relative to each other in a particular angular position of the container 40 and the cover 10 to each other. Therefore, twisting the handle 80 and the cover 10 relative to each other in the closing direction causes the container 40 to be entrained by the cover 10 via the cavity edge 12a engaging the protrusion 51a. This engagement is in addition to the engagement provided between the cover 10 and the container 40 via the second engagement mechanism (see Figures 9 and 10). The redundant engagement of the cover 10 and the container 40 with each other by independent engagement means provides for a reliable re-engaging of the first engagement mechanism. The re-engaging of the first engagement mechanism is particularly important so that the dispenser 1 automatically re-opens when the dispenser is used for dispensing material the next time.

Fig. 19 illustrates a stage at which the handle 80 and the cover 10 are further rotated toward the closing direction and at which the pawl 51 is (re-)engaged in the recess 52. At this stage also the first and second retention elements 54a, 54b are engaged with each other. In addition, the cavity 12 is displaced from the pawl 51 so that a wall of the cover 10 restricts a movement of the pawl 51 radially outwardly. This restriction of the movement of the pawl 51 locks the first and second retention elements 54a, 54b against disengagement from each other at positions outside the open position of the dispenser 1. Accordingly, a premature disengagement of the first and second retention elements 54a, 54b is prevented during opening of the dispenser until the dispenser is fully opened. This prevents dispensation of material in case the dispenser 1 is closed or only partially open.

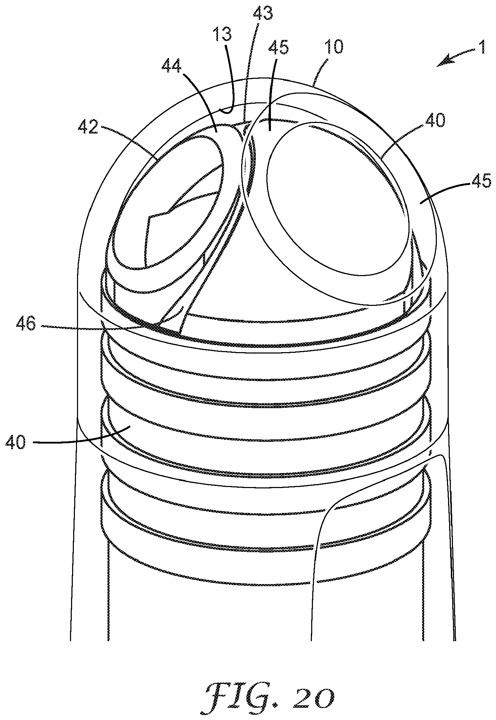

Fig. 20 shows a front portion of each the container 40 and the cover 10. The container 40 has a generally ball-shaped or bullet-shaped outer front end 43 which matches with a corresponding ball-shaped or bullet-shaped inner surface 13 of the cover 10. In the example, the outer front end 43 is recessed outside a ring-shaped seal 44 around the outlet 42. The recessed area 45 provides for a space between the cover 10 and the container 40 when the cover 10 and the container 40 are assembled with the seal 44 abutting the inner surface 13 of the cover 10. Therefore, any material that unintentionally builds up or reaches between the cover 10 and the container 40 can be at least partially absorbed in the recessed area 45. Thus, any areas between the cover 10 and the container 40 that are in direct contact with each other and that are contaminated by material can be minimized. This further helps minimizing the force required to rotate the container 40 and the cover 10 relative to each other, even with contamination of areas between the cover 10 and the container 40. Because the forces required to rotate the container 40 and the cover 10 relative to each other can affect the operation of the dispenser, in particular in connection with the impeding and/or snap-in force of the first engagement mechanism, this further helps maximizing the reliability of the dispenser.

The outer front end 43 of the container further has a notch 46 which extends from a location adjacent the front end 43 past the outlet 11 toward a rear end (opposite of the front end 43 but not visible in this view). The notch 46 is located in the container such that it extends between the outlet 42 and the dispensing opening 11 when the dispenser 1 is in the closed position. In the closed position the outlet 42 and the dispensing opening 11 are offset and spaced relative to each other and the notch 46 is arranged in the container 40 so that it is positioned in the space between the outlet 42 and the dispensing opening 11. The notch 46 thus interrupts a path between the outlet 42 and the dispensing opening 11 on which a liquid could creep (for example due to capillary effects) from the dispensing opening 11 toward the outlet 42 where the liquid may contaminate the material stored in the container 40. Such a liquid may for example include a disinfectant used for cleaning the closed device. The notch 46 may be connected to a volume that is provided between the cover 10 and the container 40 further toward the rear end of the container. Thus a relatively great amount of liquid may be guided away from the outlet 42 and absorbed in the volume.

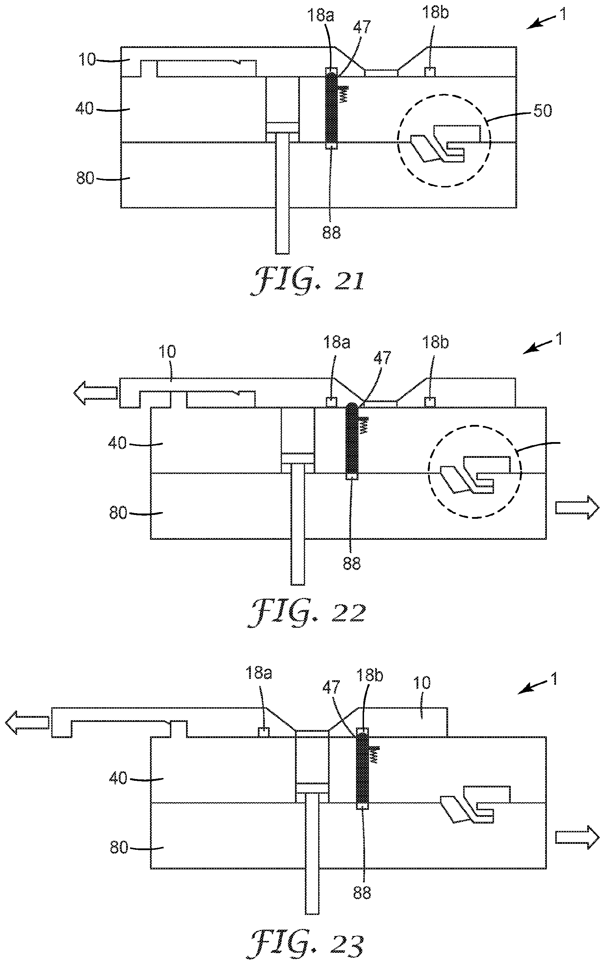

Fig. 21 shows a dispenser 1 which is identical to the embodiment illustrated in Figures 4 to 11 except that the second engagement mechanism is modified as described in the following. It is noted that all other features and functions of the embodiment shown in Figures 21-23 are identical to the embodiment illustrated in Figures 4 to 11 and therefore for the description of the other features and functions of the embodiment in Figures 21-23 the description about Figures 4 to 11 can be referred to without change.

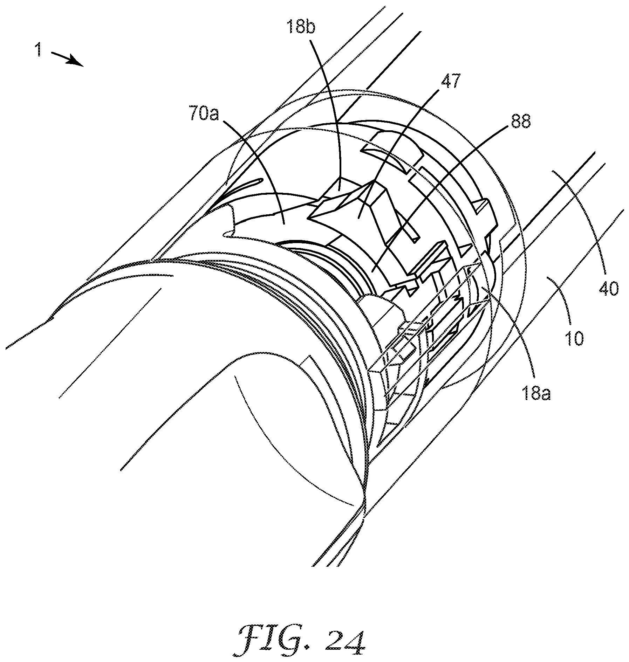

In the embodiment shown in Figures 21-23 there is no detent (as designated by numeral 24 in Figures 4 to 11). The second engagement mechanism instead has a spring cam 47. The spring cam 47 is attached at the container 40 resiliently urged toward the cover 10. It is noted that the example is very schematic for illustrating the principle features and operation of the modified second engagement mechanism. One implementation into reality is described in Figures 24 to 26.

In the example of Figures 21-23 the spring cam 47 is radially (in the Figure vertically) movable against spring force, but fixed relative to the container 40 against a movement in a circumferential direction (in the Figure horizontally). The cover 10 has a first engagement groove 18a into which the spring cam 47 engages. Accordingly, the cover 10 and the container 40 are retained against relative movement to each other. By urging the handle 80 and the cover 10 in the dispensing direction relative to each other the spring cam 47 is urged out of the first engagement groove 18a and thus disengages from the first engagement groove 18a as shown in Fig. 22. This is enabled in the example by an inclined structure, for example a chamfer or cone provided on the portion of the spring cam 47 which extends into the engagement groove 18a. Due to the inclined structure the spring cam 47 is urged radially in a direction out of the engagement groove 18a as a force in the circumferential direction is exerted on the inclined structure.

The handle 80 (or a part that is fixedly connected with the handle 80) has a second engagement groove 88. As shown in Fig. 22 the spring cam 47 moves into the second engagement groove 88 in a situation in which the snap stop 50 of the first engagement mechanism is engaged. Thereby the handle 80 and the container 40 are locked with each other against relative movement to each other. Because the spring cam 47 at the portion which extends into the engagement groove 88 does not have an inclined structure no radial force occurs upon a force in the circumferential direction is exerted by the handle 80 on the spring cam 47. This prevents that the container 40 and the handle 80 move relative to each other as long as the cover 10 is in an intermediate position between positions in which the dispenser 1 is closed or open. This further prevents situation in which material is extruded although the dispenser is not fully open.

As shown in Fig. 23 the cover 10 has a third engagement groove 18b. Upon reaching a position in which the dispenser is open the spring cam 47 snaps into the third engagement groove 18b, thus retracting from the second engagement groove 88. Accordingly, once the dispenser 1 is open the container 40 and the handle 80 are unlocked from each other and enabled for a relative movement to each other. Further, if the dispenser 1 is open the cover 10 and the container 40 are retained relative to each other via the spring cam 47 engaging the second engagement groove 18b. Like described for the retention between the spring cam 47 and the first engagement groove 18a, due to the inclined structure the retention can be overcome by urging the cover 10 relative to the container 40 in the circumferential direction. Upon closing the dispenser 1 therefore the spring cam 47 is caused to disengage from the second retention groove 18b in consequence of urging the container 40 and the cover 10 in the circumferential direction to each other.

Figures 24-26 illustrate an implementation of the principle described in Figures 21-23. Figures 24-26 correspond to the dispenser shown in Fig. 12 (for example) except that the second engagement mechanism is modified as described in the following. Fig. 24 shows the dispenser 1, having a cover 10, a container 40 and a handle 80. The dispenser 1 is generally operable as described in Fig. 3. Further, the dispenser has a spring cam 47 which in the situation shown engages a third engagement groove 18b. In the situation shown the dispenser 1 is open. Therefore, the situation shown corresponds to the situation illustrated in Fig. 23. When the dispenser is closed (not shown) the dispenser engages a first engagement groove 18a.

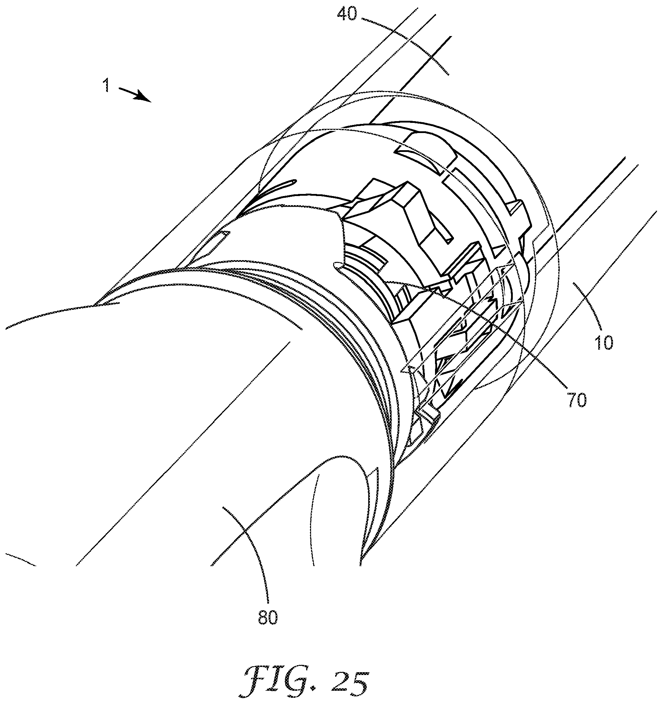

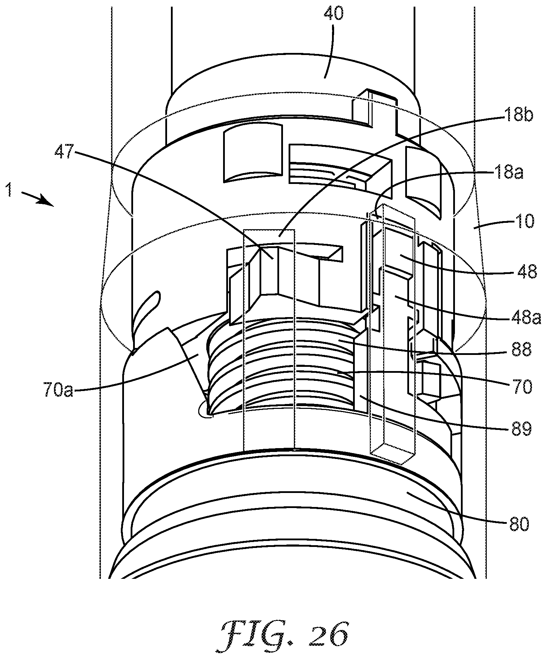

From this situation a rotation of the handle 80 and the cover 10 relative to each other in the dispensing direction causes pasty material stored in the dispenser 1 to be dispensed, as described in Fig. 7. Thereby the handle 80 and the container 40 move relative to each other so that also the screw plunger (no. 70 in Figures 25 and 26) moves relative to the container 40. In the example, the dispenser has a washer 70a which is slotted and therefore forms a second engagement groove 88. The washer in the example is mounted on the screw plunger 70 and is rotationally fixed thereon. However in another embodiment the washer 70a may be integrally molded with the screw plunger 70.

In the situation shown the spring cam 47 is enabled for a movement into the second engagement groove 88, for example in case the dispenser 1 would be closed. However, because the spring cam 47 is resiliently urged toward the cover 10, the spring cam 47 remains disengaged from the second engagement groove 88 as long as the spring cam 47 can engage into the third engagement groove 18b (or into the first engagement groove 18 a). Hence, a rotation of the handle 80 and the container 40 in the dispensing direction is enabled and after a slight rotation the situation shown in Fig. 25 is reached. In the situation shown in Fig. 25 the spring cam 47 is prevented by the washer 70a from moving out of the third engagement groove 18b. Accordingly, as long as the second engagement groove 88 and the spring cam 47 are misaligned from each other, the spring cam 47 locks the cover 10 in place. Therefore, when the second engagement groove 88 and the spring cam 47 are misaligned from each other, the cover 10 and the container 40 cannot be rotated relative to each other. This prevents that during dispensing the pasty material the dispenser 1 cannot be closed before the screw plunger 70 has somewhat retracted from the container 40. Thus, inadvertent closing the dispenser while the pasty material is still under pressure during dispensing is prevented.

There are situations in which the second engagement groove 88 and the spring cam 47 are almost aligned with each other but still slightly misaligned. During a rotation a situation may occur as illustrated in Fig. 26. In this situation the washer 70a still restricts the movement of the spring cam 47 toward the second engagement groove 88, however, due to the tongue-like design of the spring cam 47 the spring cam 47 may itself deform upon being urged toward the second engagement groove 88. Accordingly, in such a situation the cover 10 would be become rotatable relative to the container 40, which is undesired. Therefore, the container 4 has a locking mechanism 48 which locks the cover 10 in situations in which the second engagement groove 88 and the spring cam 47 are almost aligned with each other but in which they are still slightly misaligned. The locking mechanism 48 comprises a rocker 48a which is configured so that it can engage the first engagement groove 18a. The rocker 48a is movable by a circumferential control portion 89 of the handle 80 between a locking position in which the rocker 48a engages with the first engagement groove 18a and a release position in which the rocker 48a disengages from the first engagement groove 18a. In the example, the rocker 48a is set up to resiliently retract from cover 10 toward the release position. The circumferential control portion 89 of the handle 80 is configured such that the control portion 89 pushes the rocker 48a into the locking position in angular positions of the handle 80 and the container 40 in which the second engagement groove 88 and the spring cam 47 are almost aligned with each other but in which they are still slightly misaligned. The circumferential control portion 89 of the handle 80 is further configured such that the control portion 89 releases the rocker toward the release position in the other angular positions.

## Claims

1. A dispenser (1) for a pasty material comprising a container (40) for the material, a cover (10) and a handle (80),
the dispenser (1) being operable by twisting the cover (10) and the handle (80) about a rotation axis relative to each other in a dispensing direction for dispensing material and in an opposite closing direction,
the container (40) having an outlet (42) for the material and the cover (10) having a dispensing opening (11), wherein the container (40) and the cover (10) are arranged relative to each other for a rotation between a closed position, in which the cover (10) closes the outlet (42), and an open position, in which the dispensing opening (11) opens the outlet (42);
a first engagement mechanism being provided for enabling the container (40) and the handle (80) to engage with each other in at least one angular position during twisting in the closing direction, the first engagement mechanism further forming a freewheel mechanism with respect to a rotation in the dispensing direction;
the first engagement mechanism comprising a first snap-stop (50) for providing the engagement between the handle (80) and the container (40) and for impeded disengagement when engaged;
a second engagement mechanism being provided for enabling the rotation of the container (40) and the cover (10) in an angular range between the closed position and the open position and restricting a rotation outside that range;
the second engagement mechanism comprising a second snap-stop (20) for providing the restriction between the container (40) and the cover (10) with respect to a twisting toward the dispensing direction and providing for an impeded disengagement when engaged in the open position;
the first snap-stop (50) providing for an impeding force to be overcome for disengagement and the second snap-stop (20) providing for a snap-in force to be overcome for engagement;
wherein the first and second snap-stop are configured such that the impeding force is greater than the snap-in force.

2. The dispenser of claim 1, wherein the first snap-stop comprises a pawl and a receptacle within which the pawl can be received in a pre-engaged position, in which further movement of the pawl into the receptacle is enabled, and an engaged position, in which the snap-stop is engaged and further movement of the pawl into the receptacle is prevented.

3. The dispenser of claim 2, wherein the pawl is formed by a partial circumferential wall of the container around the rotation axis, and wherein the receptacle is formed in at least a partial circumferential wall of the handle around the rotation axis.

4. The dispenser of claim 2 or 3, wherein the pawl is formed by the container and the receptacle is formed by the handle.

5. The dispenser of any of the claims 2 to 4, wherein the pawl has a first retention element and the receptacle has a second retention element for engaging with each other to provide the impeding against disengagement of the first snap-stop.

6. The dispenser of claim 5, wherein the first and second retention element are configured for elastically yielding and resetting radially from the rotation axis for engaging and disengaging, wherein the cover has a cavity in a wall that faces the pawl, wherein the pawl is restricted against a radially outward movement by the wall in the closed position and such that the cavity provides a space for the pawl to radially outwardly move in the open position.

7. The dispenser of any of the claims 2 to 6, being provided with an audible click function, the click of the click function being triggered in at least one angular position during rotation of the handle and the container relative to each other in the dispensing direction.

8. The dispenser of claim 7, wherein the audible click function is provided by a baffle formed by the handle that cooperates with the pawl such that during the rotation of the handle and the container relative to each other the baffle and the pawl periodically meet, whereby the baffle elastically deflects the pawl radially inwardly and releases the pawl subsequently, wherein the click is generated in consequence of the pawl radially resetting when released.

9. The dispenser of any of the preceding claims, wherein the second engagement mechanism comprises a tappet and a detent for disengageable engagement with one another.

10. The dispenser of any of the preceding claims, further having a screw plunger, the screw plunger and the handle being anti-twist locked with each other but axially displaceable relative to each other.

11. The dispenser of any of the preceding claims, wherein the cover and the handle are attached to one another and entirely enclose the container except for any portion of the container which is exposed by the dispensing opening.

12. The dispenser of any of the preceding claims, wherein a notch is provided between the container and the cover, the notch extending over a distance in a dimension parallel the rotation axis and is arranged to be located between the outlet and the dispensing opening in the closed position.

13. The dispenser of any of the preceding claims, wherein a recessed area is provided in the container to provide a space between the container and the cover, the recessed area being located outside a ring-shaped seal around the outlet.

14. The dispenser of any of the preceding claims, wherein a third engagement mechanism is provided for impeding a rotation in the closing direction of the container and the cover relative to each other, when the dispenser is in the open position.

15. A method of dispensing a pasty material, comprising the steps of:
providing a dispenser according to any one of the preceding claims,
starting from the closed position, twisting the handle and the cover in the dispensing direction relative to each other and thereby entraining the container by the handle based on an engagement of the container and the handle with each other;
further twisting the handle and the cover in the dispensing direction relative to each other and thereby reaching the open position in which the cover and the container are engaged for impeded disengagement;
further twisting the handle and the cover in the dispensing direction relative to each other and causing the container and the handle to disengage from each other;
further twisting the handle and the cover in the dispensing direction relative to each other and thereby dispensing the material;
twisting the handle and the cover in the closing direction relative to each other whereby the handle and the container rotate relative to each other;
further twisting the handle and the cover in the closing direction relative to each other and thereby causing the container and the handle to engage with each other;
further twisting the handle and the cover in the closing direction relative to each other and thereby causing the container and the cover to disengage from each other; and
further twisting the handle and the cover in the closing direction relative to each other and thereby reaching the closed position.

## Patentansprüche

1. Ein Spender (1) für ein pastöses Material, umfassend einen Behälter (40) für das Material, eine Hülle (10) und einen Griff (80),
wobei der Spender (1) durch Verdrehen der Hülle (10) und des Griffs (80) um eine Rotationsachse relativ zueinander in eine Abgaberichtung zum Abgeben von Material und in eine entgegengesetzte Schließrichtung betätigt werden kann,
wobei der Behälter (40) einen Auslass (42) für das Material aufweist und die Hülle (10) eine Abgabeöffnung (11) aufweist, wobei der Behälter (40) und die Hülle (10) relativ zueinander für eine Drehung zwischen einer geschlossenen Position, in der die Hülle (10) den Auslass (42) verschließt, und einer offenen Position, in der die Abgabeöffnung (11) den Auslass (42) öffnet, angeordnet sind;
wobei ein erster Einrastmechanismus bereitgestellt ist, um es dem Behälter (40) und dem Griff (80) zu ermöglichen, während des Verdrehens in Schließrichtung in mindestens einer Winkelstellung ineinander einzurasten, wobei der erste Einrastmechanismus ferner einen Freilaufmechanismus in Bezug auf eine Drehung in Abgaberichtung bildet;
wobei der erste Einrastmechanismus einen ersten Schnappanschlag (50) umfasst, der das Einrasten zwischen dem Griff (80) und dem Behälter (40) bereitstellt und im eingerasteten Zustand das Ausrasten verhindert;
wobei ein zweiter Einrastmechanismus bereitgestellt ist, um die Drehung des Behälters (40) und der Hülle (10) in einem Winkelbereich zwischen der geschlossenen und der offenen Position zu ermöglichen und eine Drehung außerhalb dieses Bereichs einzuschränken;
wobei der zweite Einrastmechanismus einen zweiten Schnappanschlag (20) umfasst, um die Beschränkung zwischen dem Behälter (40) und der Hülle (10) in Bezug auf ein Verdrehen in Abgaberichtung bereitzustellen und ein Ausrasten zu verhindern, wenn sie in der offenen Position eingerastet sind;
wobei der erste Schnappanschlag (50) das Überwinden einer verhindernden Kraft für das Ausrasten bereitstellt und der zweite Schnappanschlag (20) das Überwinden einer Einschnappkraft für das Einrasten bereitstellt;
wobei der erste und der zweite Schnappanschlag so konfiguriert sind, dass die verhindernde Kraft größer als die Einschnappkraft ist.

2. Der Spender nach Anspruch 1, wobei der erste Schnappanschlag eine Sperrklinke und eine Aufnahmevorrichtung umfasst, in der die Sperrklinke in einer Voreinrastposition, in der eine weitere Bewegung der Sperrklinke in die Aufnahmevorrichtung ermöglicht wird, und einer Einrastposition, in der der Schnappanschlag eingerastet ist und eine weitere Bewegung der Sperrklinke in der Aufnahmevorrichtung verhindert wird, aufgenommen werden kann.

3. Der Spender nach Anspruch 2, wobei die Sperrklinke durch eine Teilumfangswand des Behälters um die Rotationsachse gebildet wird und wobei die Aufnahmevorrichtung in mindestens einer Teilumfangswand des Griffs um die Rotationsachse gebildet wird.

4. Der Spender nach Anspruch 2 oder 3, wobei die Sperrklinke durch den Behälter und die Aufnahmevorrichtung durch den Griff gebildet wird.

5. Der Spender nach einem der Ansprüche 2 bis 4, wobei die Sperrklinke ein erstes Rückhalteelement und die Aufnahmevorrichtung ein zweites Rückhalteelement zum Einrasten ineinander aufweist, um das Ausrasten des ersten Schnappanschlags zu verhindern.

6. Der Spender nach Anspruch 5, wobei das erste und das zweite Rückhalteelement so konfiguriert sind, dass sie zum Ein- und Ausrasten elastisch nachgeben und sich radial von der Rotationsachse zurückstellen, wobei die Hülle einen Hohlraum in einer Wand aufweist, die der Sperrklinke zugewandt ist, wobei die Sperrklinke in der geschlossenen Position gegen eine Bewegung radial nach außen durch die Wand eingeschränkt ist, und zwar so, dass der Hohlraum einen Raum für die Sperrklinke bereitstellt, um sich in der offenen Position radial nach außen zu bewegen.

7. Der Spender nach einem der Ansprüche 2 bis 6, der mit einer hörbaren Klickfunktion ausgestattet ist, wobei das Klicken der Klickfunktion in mindestens einer Winkelposition während der Drehung des Griffs und des Behälters relativ zueinander in Abgaberichtung ausgelöst wird.

8. Der Spender nach Anspruch 7, wobei die hörbare Klickfunktion durch eine durch den Griff gebildete Trennwand bereitgestellt wird, die mit der Sperrklinke so zusammenwirkt, dass während der Drehung des Griffs und des Behälters relativ zueinander die Trennwand und die Sperrklinke periodisch aufeinander treffen, wodurch die Trennwand die Sperrklinke elastisch radial nach innen auslenkt und die Sperrklinke anschließend freigibt, wobei das Klicken als Folge der radialen Rückstellung der Sperrklinke beim Freigeben erzeugt wird.

9. Der Spender nach einem der vorstehenden Ansprüche, wobei der zweite Einrastmechanismus einen Stößel und eine Feststellvorrichtung für das lösbare Einrasten ineinander umfasst.

10. Der Spender nach einem der vorstehenden Ansprüche, der ferner einen Schraubstößel aufweist, wobei der Schraubstößel und der Griff gegen Verdrehen miteinander verriegelt, aber relativ zueinander axial verschiebbar sind.

11. Der Spender nach einem der vorstehenden Ansprüche, wobei die Hülle und der Griff aneinander befestigt sind und den Behälter vollständig umschließen, mit Ausnahme eines durch die Abgabeöffnung freigelegten Abschnitts des Behälters.

12. Der Spender nach einem der vorstehenden Ansprüche, wobei eine Aussparung zwischen dem Behälter und der Hülle bereitgestellt ist, wobei die Aussparung über einen Abstand in einer Abmessung parallel zur Rotationsachse verläuft und so angeordnet ist, dass sie sich in geschlossener Position zwischen dem Auslass und der Abgabeöffnung befindet.

13. Der Spender nach einem der vorstehenden Ansprüche, wobei ein ausgesparter Bereich in dem Behälter bereitgestellt ist, um einen Raum zwischen dem Behälter und der Hülle bereitzustellen, wobei der ausgesparte Bereich außerhalb einer ringförmigen Dichtung um den Auslass herum angeordnet ist.

14. Der Spender nach einem der vorstehenden Ansprüche, wobei ein dritter Einrastmechanismus bereitgestellt ist, um eine Drehung in der Schließrichtung des Behälters und der Hülle relativ zueinander zu verhindern, wenn sich der Spender in der offenen Position befindet.

15. Ein Verfahren zur Abgabe eines pastösen Materials, umfassend die folgenden Schritte:
Bereitstellen eines Spenders nach einem der vorstehenden Ansprüche,
ausgehend von der geschlossenen Position, Verdrehen des Griffs und der Hülle in Abgaberichtung relativ zueinander und dadurch Mitnehmen des Behälters durch den Griff aufgrund des Einrastens des Behälters und des Griffs ineinander;
weiteres Verdrehen des Griffs und der Hülle in Abgaberichtung relativ zueinander und dadurch Erreichen der offenen Position, in der die Hülle und der Behälter so eingerastet sind, dass Ausrasten verhindert wird;
weiteres Verdrehen des Griffs und der Hülle in Abgaberichtung relativ zueinander, wodurch der Behälter und der Griff sich voneinander lösen;
weiteres Verdrehen des Griffs und der Hülle in Abgaberichtung relativ zueinander und dadurch Abgeben des Materials;
Verdrehen des Griffs und der Hülle in Schließrichtung relativ zueinander, wodurch sich der Griff und der Behälter relativ zueinander drehen;
weiteres Verdrehen des Griffs und der Hülle in Schließrichtung relativ zueinander, wodurch der Behälter und der Griff ineinander einrasten;
weiteres Verdrehen des Griffs und der Hülle in Schließrichtung relativ zueinander und dadurch Lösen des Behälters und der Hülle voneinander und weiteres Verdrehen des Griffs und der Hülle in Schließrichtung relativ zueinander und damit Erreichen der geschlossenen Position.

## Revendications

1. Distributeur (1) pour une matière pâteuse comprenant un contenant (40) pour la matière, une enveloppe (10) et une poignée (80),
le distributeur (1) pouvant fonctionner par torsion de l'enveloppe (10) et de la poignée (80) autour d'un axe de rotation l'un par rapport à l'autre dans une direction de distribution pour distribuer la matière et dans une direction opposée de fermeture,
le contenant (40) ayant une sortie (42) pour la matière et le couvercle (10) ayant une ouverture de distribution (11), dans lequel le contenant (40) et le couvercle (10) sont disposés l'un par rapport à l'autre pour une rotation entre une position fermée, dans laquelle l'enveloppe (10) ferme la sortie (42), et une position ouverte, dans lequel l'ouverture de distribution (11) ouvre la sortie (42) ;
un premier mécanisme de mise en prise étant prévu pour permettre au contenant (40) et à la poignée (80) de venir en prise l'un avec l'autre dans au moins une position angulaire pendant la torsion dans la direction de fermeture, le premier mécanisme de mise en prise formant en outre un mécanisme de roue libre par rapport à une rotation dans la direction de distribution ;
le premier mécanisme de mise en prise comprenant une première butée d'encliquetage (50) pour assurer la mise en prise entre la poignée (80) et le contenant (40) et pour une libération entravée lorsqu'il est en prise ;
un deuxième mécanisme de mise en prise étant prévu pour permettre la rotation du contenant (40) et de l'enveloppe (10) dans une plage angulaire entre la position fermée et la position ouverte et limiter une rotation à l'extérieur de cette plage ;
le deuxième mécanisme de mise en prise comprenant une deuxième butée d'encliquetage (20) pour fournir la restriction entre le contenant (40) et l'enveloppe (10) par rapport à une torsion vers la direction de distribution et pour fournir une libération entravée lorsqu'il est en prise dans la position ouverte ;
la première butée d'encliquetage (50) fournissant une force d'entrave à surmonter pour la libération et la deuxième butée d'encliquetage (20) fournissant une force d'encliquetage à surmonter pour la mise en prise ;
dans lequel les première et deuxième butées d'encliquetage sont configurées de telle sorte que la force d'entrave est supérieure à la force d'encliquetage.

2. Distributeur selon la revendication 1, dans lequel la première butée d'encliquetage comprend un cliquet et un réceptacle à l'intérieur duquel le cliquet peut être reçu dans une position pré-mise en prise, dans lequel un mouvement supplémentaire du cliquet dans le réceptacle est permis, et une position en prise, dans laquelle la butée d'encliquetage est en prise et un mouvement supplémentaire du cliquet dans le réceptacle est empêché.

3. Distributeur selon la revendication 2, dans lequel le cliquet est formé par une paroi circonférentielle partielle du contenant autour de l'axe de rotation, et dans lequel le réceptacle est formé dans au moins une paroi circonférentielle partielle de la poignée autour de l'axe de rotation.

4. Distributeur selon la revendication 2 ou 3, dans lequel le cliquet est formé par le contenant et le réceptacle est formé par la poignée.

5. Distributeur selon l'une quelconque des revendications 2 à 4, dans lequel le cliquet a un premier élément de retenue et le réceptacle a un second élément de retenue pour venir en prise l'un avec l'autre pour assurer l'entrave contre la libération de la première butée d'encliquetage.

6. Distributeur selon la revendication 5, dans lequel les premier et second éléments de retenue sont configurés pour une déformation élastique et une réinitialisation radiale à partir de l'axe de rotation pour la mise en prise et la libération, dans lequel l'enveloppe comporte une cavité dans une paroi, qui est tournée vers le cliquet, dans lequel le cliquet est limité concernant un mouvement radialement vers l'extérieur par la paroi dans la position fermée et de telle sorte que la cavité fournit un espace pour que le cliquet se déplace radialement vers l'extérieur dans la position ouverte.

7. Distributeur selon l'une quelconque des revendications 2 à 6, doté d'une fonction de clic audible, le clic de la fonction de clic étant déclenché dans au moins une position angulaire pendant la rotation de la poignée et du contenant l'un par rapport à l'autre dans la direction de distribution.

8. Distributeur selon la revendication 7, dans lequel la fonction de clic audible est fournie par un déflecteur formé par la poignée, qui coopère avec le cliquet de telle sorte que durant la rotation de la poignée et du contenant l'un par rapport à l'autre, le déflecteur et le cliquet se rencontrent périodiquement, moyennant quoi le déflecteur dévie élastiquement le cliquet radialement vers l'intérieur et libère le cliquet ultérieurement, dans lequel le clic est généré suite à la réinitialisation radiale du cliquet lors de sa libération.

9. Distributeur selon l'une quelconque des revendications précédentes, dans lequel le deuxième mécanisme de mise en prise comprend un poussoir et une détente pour une mise en prise débrayable l'un avec l'autre.

10. Distributeur selon l'une quelconque des revendications précédentes, comprenant en outre un piston à vis, le piston à vis et la poignée étant bloqués contre la torsion l'un avec l'autre mais déplaçables axialement l'un par rapport à l'autre.

11. Distributeur selon l'une quelconque des revendications précédentes, dans lequel l'enveloppe et la poignée sont fixées l'un à l'autre et enserrent entièrement le contenant à l'exception de toute partie du contenant qui est exposée par l'ouverture de distribution.

12. Distributeur selon l'une quelconque des revendications précédentes, dans lequel une encoche est prévue entre le contenant et l'enveloppe, l'encoche s'étendant sur une distance dans une dimension parallèle à l'axe de rotation et étant agencée pour être située entre la sortie et l'ouverture de distribution dans la position fermée.

13. Distributeur selon l'une quelconque des revendications précédentes, dans lequel une zone en retrait est prévue dans le contenant pour fournir un espace entre le contenant et l'enveloppe, la zone en retrait étant située à l'extérieur d'un joint en forme d'anneau autour de la sortie.

14. Distributeur selon l'une quelconque des revendications précédentes, dans lequel un troisième mécanisme de mise en prise est prévu pour empêcher une rotation dans la direction de fermeture du contenant et de l'enveloppe l'un par rapport à l'autre, lorsque le distributeur est dans la position ouverte.

15. Procédé de distribution d'une matière pâteuse, comprenant les étapes consistant à :
fournir un distributeur selon l'une quelconque des revendications précédentes,
en partant de la position fermée, faire tourner la poignée et l'enveloppe dans la direction de distribution l'un par rapport à l'autre et entraîner de ce fait le contenant par la poignée sur la base d'une mise en prise du contenant et de la poignée l'un avec l'autre ;
encore faire tourner la poignée et l'enveloppe dans la direction de distribution l'un par rapport à l'autre et ainsi atteindre la position ouverte dans laquelle l'enveloppe et le contenant sont en prise pour une libération entravée ;
encore faire tourner la poignée et l'enveloppe dans la direction de distribution l'un par rapport à l'autre et amener le contenant et la poignée à se libérer l'un de l'autre ;
encore faire tourner la poignée et l'enveloppe dans la direction de distribution l'un par rapport à l'autre et pour ainsi distribuer la matière ;
faire tourner la poignée et l'enveloppe dans la direction de fermeture l'un par rapport à l'autre moyennant quoi la poignée et le contenant tournent l'un par rapport à l'autre ;
encore faire tourner la poignée et l'enveloppe dans la direction de fermeture l'un par rapport à l'autre et amener ainsi le contenant et la poignée à venir en prise l'un avec l'autre ;
encore faire tourner la poignée et l'enveloppe dans la direction de fermeture l'un par rapport à l'autre et amener ainsi le contenant et l'enveloppe à se libérer l'un de l'autre ; et encore faire tourner la poignée et l'enveloppe dans la direction de fermeture l'un par rapport à l'autre et ainsi atteindre la position fermée.
